# EUROPÄISCHE PATENTSCHRIFT

(11) **EP 2 615 121 B1**
(45) Veröffentlichungstag und Bekanntmachung des Hinweises auf die Patenterteilung: **19.11.2014**
(21) Anmeldenummer: 13151005.9
(22) Anmeldetag: 11.01.2013
(51) Int. Cl.: C08F 220/36

(54) **Zusammensetzung und Verfahren zur Korrektur eines Spaltnagels**
Composition and method for correcting a split nail
Composition et procédé destinés à la correction d'un ongle fendu

(30) Priorität: 12.01.2012 DE 102012200428
(43) Veröffentlichungstag der Anmeldung: 17.07.2013
(73) Patentinhaber: Hillebrand, Christa, 83700 Rottach (DE)
(72) Erfinder: Hillebrand, Christa, 83700 Rottach (DE)
(74) Vertreter: Franke, Dirk

(56) Entgegenhaltungen:
- EP-A2- 0 391 322
- DE-A1- 10 144 531
- US-A1- 2005 032 927

## Beschreibung

### Technisches Gebiet

Die Erfindung betrifft eine Zusammensetzung, umfassend mindestens ein erstes strahlenhärtbares Polyurethanacrylat, mindestens einen ersten Ester, und mindestens einen zweiten Ester, wobei der erste Ester ein Methacrylatester ist und der zweite Ester ein multifunktioneller Acrylatester ist, und wobei die Zusammensetzung eine Polymerisationsschrumpfung gemessen nach 24 Stunden von größer oder gleich 2 %, vorzugsweise größer oder gleich 4 % und insbesondere größer oder gleich 5 % aufweist, sowie ein Verfahren unter Verwendung der Zusammensetzung.

Ferner betrifft die vorliegende Erfindung ein Kombinationspräparat zum Aufbringen auf eine Nagelplatte, umfassend mindestens eine Zusammensetzung 1 und eine Zusammensetzung 2, wobei die Zusammensetzung 1 mindestens ein erstes strahlenhärtbares Polyurethanacrylat, mindestens einen ersten Ester und , mindestens einen zweiten Ester umfasst, wobei der mindestens eine erste Ester ein Methacrylatester ist und wobei der mindestens eine zweite Ester ein multifunktioneller Acrylatester ist, und wobei die Zusammensetzung 2 mindestens ein zweites strahlenhärtbares Polyurethanacrylat, mindestens einen multifunktionellen Polyester und mindestens einen Methacrylatester umfasst, die Verwendung dieses Kombinationspräparates zur kosmetischen Korrektur eines Spaltnagels sowie eine polymerisierte Grundmasse herstellbar aus dem Kombinationspräparat.

### Hintergrund der Erfindung

Eine Brüchigkeit der Nägel ist neben der durch Hefen oder Schimmelpilze ausgelösten Onychomykose die häufigste Erkrankung der Nägel. Ein sogenannter Spaltnagel tritt dabei in verschiedenen Formen auf, die sich hinsichtlich ihrer Ursache, Ausprägung und Korrekturmöglichkeiten voneinander unterscheiden. Man differenziert zwischen singulärer und multipler Längsfurchung und -spaltung (Onychorrhexis), multipler zinnenförmiger Spaltung, der Transversalspaltung, der lamellaren Spaltung (Onychoschizia) und der allgemeinen Nagelbrüchigkeit. Ursächlich sind zumeist externe physikalische oder chemische Noxen, aber auch systemische Störungen (wie z. B. Eisenmangel und eine damit verbundene Anämie, vaskuläre Störungen und endokrinologische Störungen wie Hyperthyreose oder Hypoparathyreoidismus) können eine Nageldystrophie hervorrufen, die mit erhöhter Brüchigkeit der Nägel einhergeht.

Zur Korrektur werden verschiedene Verfahren verwendet.

Zum einen zielen Maßnahmen auf die Beseitigung der auslösenden Ursache. So kann zum Beispiel einer durch externe Faktoren (v.a. durch starke Reinigungsmittel) ausgelösten Nagelbrüchigkeit durch das einfache Tragen von Baumwollinnenhandschuhen entgegengewirkt werden. Eine effektive Korrektur systemischer Störungen kann eine damit einhergehende Nageldystrophie beseitigen.

Zum anderen richten sich Konzepte direkt auf die Behandlung des Nagels. Verfahren zur Korrektur von brüchigen Nägeln und Spaltnägeln, welche am Nagel selbst angreifen, sind aus dem Stand der Technik bekannt. Zum einen kann auf den geschädigten Nagel eine Nagelprothese (künstlicher Fingernagel) oder eine Teilprothese (Unterstützer) aufgeklebt werden. So wird in EP 326288 A1 ein Mittel zur Reparatur eines gespaltenen oder gebrochenen Fingernagels offenbart, wobei eine künstliche Nagelplatte derart um den Nagelrand und auf diesen aufgebracht sowie mit diesem verklebt wird, dass ein Teil der künstlichen Nagelplatte unter dem Nagelrand und ein Teil über dem Nagelrand nach Art eines Nagelauslegers zu liegen kommt.

Andere Verfahren basieren auf der Verwendung spezieller Nagellacke, welche den Nagel entweder härten sollen oder als Vehikel für bestimmte Substanzen dienen, die eine Verbesserung der Struktur des Nagels herbeiführen sollen. Die Druckschrift WO 0226199 A offenbart einen Nagellack, welcher aus Rohstoffen rein pflanzlichen oder im Falle des Schellacks rein pflanzlich-tierischen Ursprungs ist. Der Nagellack wird unter Verwendung von reinem Ethylalkohol als Lösungsmittel und Schellack in Kombination mit Benzoe-Öl und/oder anderen Baumharzen und/oder ätherischen Ölen hergestellt. In einer Reihe von Druckschriften werden Härter und härtende Zusätze wie Formaldehyd (Formol; US 4,179,304; US 2008/0145325 A1; Baran and Schoon, 2004), Harnstoff (Baran and Schoon, 2004), Bakterienextrakt (US 2005/0006888 A), Citral (3,7-dimethyl-2,6-octadienal; US 2004/0170584 A1), Glycerin-Wasser Gemisch (US 2006/0057082 A), kristalline anorganische Fasern (DE 69111621 T2), und Chitin (DE 3 537 333 C2) beschrieben.

Alle angeführten Zusammensetzungen und Verfahren weisen den Nachteil auf, dass der fortschreitenden Rissbildung nicht entgegengewirkt wird, sondern lediglich ein (oftmals nur geringer) Härtungseffekt erzielt wird. Im Falle künstlicher Nägel sind diese zudem auch umständlich auf die Nagelplatte aufzubringen.

Nagellacke auf der Basis künstlicher Polymere sind seit langem bekannt, vor allem solche Nagellacke, in denen leichtflüchtige organische Lösungsmittel zur Anwendung kommen, z. B. US 6123931 A1. Neuere Nagellacke auf Polymerbasis verzichten weitgehend oder vollständig auf umweltbelastende oder gesundheitsschädliche Lösungsmittel. Es ist beispielsweise aus der Anmeldung EP 418 469 bekannt, eine wässrige Polyurethandispersion allein oder in Kombination mit einer wässrigen Dispersion von Vinylestern und/oder Acrylestern in Nagellacken zu verwenden. Ein Polyurethan in wässriger Dispersion ist ferner aus der Anmeldung EP 391 322 für eine kosmetische Zusammensetzung vom Nagellack-Typ bekannt, wobei als Filmbildner eine wässrige Dispersion eines Polyurethanpolymers oder eines Polyurethan/Acrylstyrol-Copolymers verwendet wird. In den Druckschriften EP 0 143 480 A, EP 0 418 469 A, EP 0 593 959 A und EP 0 628 304 A werden ferner Nagellack-Zusammensetzungen auf der Basis einer wässrigen Polyurethandispersion und einer Acryldispersion beschrieben, welche einen gut auf dem Nagel haftenden, aber nach Art eines Überzugs abziehbaren Lackfilm ermöglichen sollen. Polymerüberzüge auf der Basis einer wässrigen Dispersion von Polyurethanen werden in der WO 93/18075 A1 beschrieben.

Keine der vorgenannten Druckschriften stellt eine Zusammensetzung bereit, die hinreichend gut auf einer Nagelplatte haftet und gleichzeitig genügend Polymerisationschrumpfung aufweist, um eine kosmetische Korrektur eines Spaltnagels zu ermöglichen.

Aufgabe der vorliegenden Erfindung ist es daher, eine Zusammensetzung und/oder ein Kombinationspräparat bereitzustellen, welche/s eine hervorragende Haftung auf einer Nagelplatte und gleichzeitig genügend Polymerisationsschrumpfung aufweist, um eine kosmetische Korrektur eines Spaltnagels zu ermöglichen, ohne jedoch zur gleichen Zeit zu einem Austrocknen oder Verspröden des Nagels zu führen. Ferner soll die Zusammensetzung auf umweltschädliche und/oder gesundheitsbelastende Inhaltsstoffe, z. B. Lösungsmittel, verzichten. Das erfindungsgemäße Verfahren bzw. die erfindungsgemäße Verwendung zur kosmetischen Korrektur eines Spaltnagels soll zudem einfach auszuführen sein bei gleichzeitig möglichst geringer Applikationshäufigkeit der erfindungsgemäßen Zusammensetzung und/oder des Kombinationspräparats.

Gelöst wird diese Aufgabe durch die erfindungsgemäße Zusammensetzung, das erfindungsgemäße Verfahren, das erfindungsgemäße Kombinationspräparat und die erfindungsgemäßen Verfahren gemäß der beiliegenden Patentansprüche. Die Unteransprüche betreffen zudem bevorzugte Ausgestaltungen der Erfindung.

### Detaillierte Beschreibung der Erfindung

In einem ersten Aspekt betrifft die vorliegende Erfindung daher eine Zusammensetzung, welche mindestens ein erstes strahlenhärtbares Polyurethanacrylat umfasst. Ferner umfasst die Zusammensetzung mindestens einen ersten Ester, wobei der erste Ester ein Methacrylatester ist, und mindestens einen zweiten Ester, wobei der zweite Ester ein multifunktioneller Acrylatester ist. Die Zusammensetzung weist ferner eine Polymerisationsschrumpfung gemessen nach 24 Stunden, von größer oder gleich 2 %, vorzugsweise größer oder gleich 4 % und insbesondere größer oder gleich 5% auf.

Unter einem Polymer wird erfindungsgemäß ein Makromolekül verstanden, welches aus gleichen oder gleichartigen Einheiten, den sogenannten Monomeren, aufgebaut ist. Man unterschiedet zwischen Polymeren, welche eine einzige Art von Monomer enthalten, den Homopolymeren (z. B. Polyethylen, Polypropylen, Polyvinylchlorid oder Polyamid PA6), und Polymeren, welche aus verschiedenen Monomeren aufgebaut sind, den Copolymeren.(z. B. Polyester, Polyurethane und auch einige Polyamide (PA 66)). Die verschiedenen Monomerarten der Copolymere können dabei in zufälliger oder sequentieller Reihenfolge auftreten, z. B. in Blöcken oder nach anderen Prinzipien geordnet sein.

Unter Polymerisation wird die Reaktion von Monomereinheiten unter Auflösung der Mehrfachbindung zum Polymer verstanden. Zum Teil erfolgt die Polymerisation unter Mithilfe von Katalysatoren. Mechanistisch kann eine Polymerisation durch verschiedene Prozesse erfolgen: man unterscheidet Stufenwachstumsreaktionen und Kettenwachstumsreaktionen. Während bei der Stufenwachstumsreaktion bereits vorhandene Kettensegmente zu einer größeren Kette zusammenwachsen können (z. B. bei der Polyaddition und Polykondensation), lagert sich bei der Kettenwachstumsreaktion immer ein Molekül nach dem anderen an das sogenannte reaktive Kettenende an. Zu den Kettenwachstumsreaktionen gehören radikalische Polymerisation, die ionische (anionische und kationische) Polymerisation und Koordinationspolymerisation.

Unter Strahlenhärtung wird ein Verfahren verstanden, bei welchem mit Hilfe von Strahlung reaktive Materialien von einem höhermolekularen Zustand überführt werden. Als Aktivierungsenergiestrahlen eignen sich elektromagnetische Strahlung und Elektronenstrahlen, wobei letztere wegen des hohen technischen Aufwands und den damit verbundenen hohen Kosten selten eingesetzt werden (z. B. zur Induktion statistischer Vernetzungen in strahlenvernetztem Polyethylen (XPE)). Elektromagnetische Strahlen werden bevorzugt im UV Bereich eingesetzt; bei Zusatz geeigneter Initiatoren können sich jedoch auch sichtbares Licht und Mikrowellen eignen.

Die UV Lichthärtung beruht generell auf zwei Reaktionsmechanismen, der radikalischen und der kationischen Polymerisation. Im kommerziellen Bereich kommen vorwiegend Verfahren zum Einsatz, welche auf einer radikalischen Polymerisation basieren, da durch diese Polymerisationsart eine hohe Aushärtungsrate der Zusammensetzung erreicht wird. Ferner kann eine große Vielzahl unterschiedlicher Monomere und Oligomere verwendet werden, wodurch die Herstellung ausgehärteter Produkte mit verschiedenen Eigenschaften ermöglicht wird. In den meisten Fällen handelt es sich bei den Ausgangsmaterialien um polyfunktionelle Acrylate, seltener um ungesättigte Polyester(harze), welche mit einem Aktivierungsenergiestrahl, wie UV-Strahlung, in der Gegenwart eines für die radikalische Photopolymerisation geeigneten Initiators ausgehärtet werden. Bei der kationischen Photoaushärtungstechnik wird ein Monomer oder ein Oligomer, welches keine Acryl-, sondern eine Epoxidverbindung oder eine Oxetanverbindung ist, mit einem Aktivierungsenergiestrahl, wie UV-Strahlung, in der Gegenwart eines kationischen Photopolymerisationsinitiators ausgehärtet. Im Gegensatz zur radikalischen Polymerisation steht für die kationische Polymerisation nur eine begrenzte Zahl von Ausgangsmaterialien zur Verfügung, weshalb die Vielzahl der so hergestellten ausgehärteten Produkte im Vergleich zum ersten Verfahren niedriger ist.

Für die UV Lichthärtung können als Strahlungsquellen üblicherweise spezielle UV-Lampen verwendet werden, wobei zwischen dotierten (z.B. Blei, Eisen, Gallium oder Thallium) und undotierten Lampen (Quecksilberdampflampe) unterschieden wird. Gasentladungslampen stellen dabei ein relativ weites Strahlenspektrum bereit, welches von UVB-Strahlung bis zum weißen Licht reicht. Auch Hochleistungs-UV LED können vorteilhaft zur Härtung genutzt werden. Die Aushärtung erfolgt im Wellenlängenbereich zwischen 320 und 450 nm. Die Lichtintensität der Quelle liegt bei 100-3000 mW/cm².

Der Begriff "multifunktionell" bezeichnet erfindungsgemäß eine chemische Substanz mit mehr als zwei funktionellen Gruppen, er ist also mindestens trifunktionell. Dabei wird unter einer funktionellen Gruppe diejenige Atomgruppe in organischen Verbindungen verstanden, die die Stoffeigenschaften und das Reaktionsverhalten der sie tragenden Verbindungen maßgeblich bestimmt.

Unter der Polymerisationsschrumpfung wird erfindungsgemäß die Schrumpfung verstanden, die während der Abbindereaktion von Polymeren stattfindet, d.h. der durch energiereiche Strahlung erzeugten Polymerisationsreaktion. Mechanistisch liegt der Schrumpfung die Ausbildung kovalenter Bindungen zwischen den Monomereinheiten bei der Polymerisationsreaktion zugrunde, wobei die so verbundenen Oligomere weniger Platz beanspruchen als frei bewegliche Monomereinheiten. Aus der Polymerisation resultiert damit eine Dichteänderung des polymerisierten Materials im Vergleich zum Ausgangszustand vor der Polymerisation. Die mit der Polymerisation verbundenen Änderungen der Materialeigenschaften können über verschiedene Techniken gemessen werden.

Zu den Techniken zur Schrumpfungsmessung gehören beispielsweise die Pyknometrie, die Dilatometrie, die Absorptionsmessung für Polymerfilme oder beispielsweise in ASTM oder ISO Standards festgelegte Messtechniken. Bevorzugt kann der in ISO 1183 oder in ASTM D792 - 08 festgelegte Standard zur Bestimmung der Dichte und der spezifischen Dichte verwendet werden. Die Zusammensetzung weist nach der Härtung vorzugsweise eine spezifische Dichte auf von 0,095 g/cm³ bis 1,2 g/cm³, besonders bevorzugt ist eine spezifische Dichte von 1,0 g/cm³ bis 1,1 g/cm³. Im Zusammenhang mit der vorliegenden Erfindung wird eine geeignete Messmethode so gewählt, dass komplexe physiologische Parameter des Nagels berücksichtigt werden können. Daher können zur Ermittlung der Polymerisationsschrumpfung auf einem Nagel humane Nagelabschnitte oder Nagelähnliche Substrate verwendet werden (Darstellung in Beispiel 2).

Die erfindungsgemäße Zusammensetzung ermöglicht eine hohe Quervernetzung der Basiskomponente, dem ersten strahlenhärtbaren Polyurethanacrylat, mit dem zweiten Ester der Zusammensetzung, dem multifunktionellen Acrylatester. Die durch UV-Lichthärtung erzeugbare hohe Quervernetzung führt vorteilhaft zu einer Schrumpfung der Zusammensetzung auf der Nagelplatte und führt so zu einem Zusammenziehen des Risses oder der Risse des Spaltnagels. Damit steht die Zusammensetzung im direkten Gegensatz zu herkömmlichen kosmetischen Nagellacken auf Polyurethanbasis, welche eine derartige Schrumpfung zu vermeiden suchen.

In einer bevorzugten Ausführungsform der Zusammensetzung kann das mindestens eine erste strahlenhärtbare Polyurethanacrylat aus der Gruppe der aliphatischen multifunktionellen Polyurethanetheracrylate und/oder der Polyurethanesteracrylate ausgewählt sein. Besonders bevorzugt ist das erste strahlenhärtbare Polyurethanacrylat ein tetra- bis octafunktionelles Polyurethanacrylat, insbesondere ein penta- bis heptafunktionelles Polyurethanacrylat, besonders bevorzugt ein hexafunktionelles Polyurethanacrylat, welches infolge seiner Vielzahl von funktionellen Gruppen hervorragende Vernetzungseigenschaften aufweist und sich überdies gut in einer wässrigen Dispersion verarbeiten lässt. Die hervorragenden Vernetzungseigenschaften bedingen letztlich auch die guten Schrumpfungseigenschaften des Copolymerisats.

Bezüglich seiner Viskosität kann das mindestens eine erste strahlenhärtbare Polyurethanacrylat so ausgestaltet sein, dass die dynamische Viskosität von 100 bis 2.000 mPa·s beträgt, bevorzugt von 200 bis 1.500 mPa·s, besonders bevorzugt von 350 bis 1.000 mPa·s. Ein derartiges aliphatisches Polyurethanacrylat ist ausreichend flexibel, so dass gute bis sehr gute filmbildende Eigenschaften vorliegen. Es ist zudem hinreichend viskös, um ein einfaches Aufbringen auf eine Nagelplatte zu ermöglichen.

Vorzugsweise kann das erste strahlenhärtbare Polyurethanacrylat in einer Molmassenverteilung vorliegen, deren Zahlenmittel der Molmasse in einem Bereich von 400 bis 1.200 g/mol angeordnet ist. Dabei bezeichnet die Molmassenverteilung für einen bestimmten Stoff die Verteilung, d.h. die anteilsmäßige Aufteilung der molaren Masse der enthaltenen Moleküle. Besonders bevorzugt kann das Zahlenmittel der Molmasse in einem Bereich von 700 bis 900 g/mol angeordnet sein. Ein derartiges aliphatisches multifunktionelles Polyurethanacrylat kann in Kombination mit einem geeigneten Vernetzer ausgezeichnete und gut steuerbare Schrumpfungseigenschaften des Copolymerisats aufweisen.

In einer weiteren Implementierung der Zusammensetzung kann der mindestens eine erste Methacrylatester ausgewählt sein aus der Gruppe bestehend aus Ethylmethacrylat, Propylmethacrylat, Butylmethacrylat, Hexylmethacrylat, Cyclohexylmethacrylat, Isodocylmethacrylat, Laurylmethacrylat, Stearylmethacrylat, Isobornylmethacrylat, Phenylmethacrylat, Benzylmethacrylat, Propylenmethacrylat, Glycolmethacrylat, Tetrahydrofurfurylmethacrylat, Hydroxyethylmethacrylat und Hydroxypropylmethacrylat. Besonders bevorzugt ist Hydroxyethylmethacrylat. Insbesondere Hydroxyethylmethacrylat weist, im Gegensatz zu anderen Methacrylatestern, nur sehr geringe allergene Eigenschaften auf und ist hydrophil, wodurch der Wasserhaushalt des Nagels vorteilhaft beeinflusst wird, da eine Reduktion des Nagel-Wassers unter den normalen Wassergehalt von ca. 15 Gew.-% die Brüchigkeit des Nagels infolge Austrocknung und Versprödung verstärkt. Insbesondere ist eine hinreichende Hydrophilie in Bezug auf Spaltnägel vorteilhaft, da die Bildung von Rissen und Spalten durch sich wiederholende Zyklen von Wasseraufnahme und Austrocknen des Nagels verstärkt wird.

Der mindestens eine multifunktionelle Acrylatester kann ausgewählt sein aus der Gruppe der dendritischen, der verzweigten oder hochverzweigten aliphatischen Polymer-Polyole. Beispielsweise kann der multifunktionelle Acrylatester ausgewählt sein aus der Gruppe, bestehend aus Diethylenglycoldi(meth)acrylat, Decandioldi(meth)acrylat, Trimethylpropan-tri(meth)acrylat, Pentaerythrittetra(meth)acrylat, Butandioldi(meth)acrylat, Ditrimethylolpropantetraacrylat, Dipentaerythrittetraacrylat, Dipentaerythritolpenta/hexaacrylat. Bevorzugt ist Pentaerythrittetraacrylat. Insbesondere Pentaerythrittetraacrylat kann eine ausreichende Schrumpfung nach UV-Lichthärtung ermöglichen, die zum Zusammenziehen des Risses oder der Risse des Spaltnagels führt, ohne jedoch eine so starke Schrumpfung zu verursachen, dass sich das Polymerisat nach der Härtung leicht von der Nagelplatte löst.

Besonders bevorzugt kann die Zusammensetzung ein strahlenhärtbares hexafunktionelles aliphatisches Polyurethan und mindestens einen ersten Ester und mindestens einen zweiten Ester umfassen, wobei der mindestens eine erste Ester vorzugsweise Hydroxyethylmethacrylat ist, und der mindestens eine zweite Ester vorzugsweise Pentaerythrittetraacrylat ist.

In einer bevorzugten Ausführungsform der Zusammensetzung kann das mindestens eine erste strahlenhärtbare Polyurethanacrylat in einer Konzentration von 1 bis 80 Gew.-% bezogen auf das Gesamtgewicht der Zusammensetzung in der Zusammensetzung enthalten sein, vorzugsweise im Bereich von 10 bis 70 Gew.-%, besonders bevorzugt im Bereich von 20 bis 65 Gew.-%, insbesondere bevorzugt von 30 bis 60 Gew.-%. Der mindestens eine erste Ester kann in einer Konzentration von 1 bis 30 Gew.-% bezogen auf das Gesamtgewicht der Zusammensetzung in der Zusammensetzung vorliegen, vorzugsweise im Bereich von 5 bis 25 Gew.-%, besonders bevorzugt im Bereich von 10 bis 20 Gew.-%. Der mindestens eine multifunktionelle Acrylatester kann in einer Konzentration von 1 bis 50 Gew.-% bezogen auf das Gesamtgewicht der Zusammensetzung in der Zusammensetzung enthalten sein, vorzugsweise im Bereich von 5 bis 45 Gew.-%, besonders bevorzugt im Bereich von 10 bis 40 Gew.-%.

Besonders bevorzugt kann die Zusammensetzung ein strahlenhärtbares Polyurethan, mindestens einen ersten Ester und mindestens einen zweiten Ester umfassen, wobei das strahlenhärtbare Polyurethan in einer Konzentration von 30 bis 60 Gew.-% bezogen auf das Gesamtgewicht in der Zusammensetzung enthalten ist, der mindestens eine erste Ester in einer Konzentration von 10 bis 20 Gew.-% bezogen auf das Gesamtgewicht in der Zusammensetzung enthalten ist, und der mindestens eine zweite Ester in einer Konzentration von 10 bis 40 Gew.-% bezogen auf das Gesamtgewicht in der Zusammensetzung enthalten ist.

Durch die so ausgestaltete Zusammensetzung werden die Eigenschaften der hervorragenden Aufbringbarkeit auf den Nagel vor dem Aushärten, sowie einer ausgezeichneten Härte und Kratzfestigkeit nach dem Aushärten miteinander verbunden. Ferner ist der Polymerisationsschrumpf ausgezeichnet geeignet, zum Zusammenziehen des Risses oder der Risse des Spaltnagels zu führen, ohne jedoch eine so starke Schrumpfung zu verursachen, dass sich das Polymerisat nach der Härtung leicht von der Nagelplatte löst.

In einer weiteren Ausführungsform kann die Zusammensetzung mindestens einen Photoinitiator umfassen. Unter einem Photoinitiator wird dabei eine chemische Substanz verstanden, die bei Lichtzutritt Strahlung absorbieren kann, um in reaktive Bestandteile zu zerfallen (meist Radikale), die ihrerseits beispielsweise eine Polymerisationsreaktion bewirken können. Im Zusammenhang mit der vorliegenden Erfindung kann durch Einsatz von Photoinitiatoren eine energieärmere anstelle einer energiereichen Strahlung verwendet werden. So können z.B. UV-Licht oder sogar sichtbares Licht (Blaulicht) verwendet werden.

Bekannte Photoinitiatoren umfassen beispielsweise Wasserstoff-Abstraktions-Radikalerzeuger, Benzophenonderivate, Acetophenonderivate, Monoacylphosphinoxide, Bisacylphosphinoxide oder Campherchinonamin-Phopolymerisationsinitiatoren. Der mindestens eine Photoinitiator kann eine Einzelverbindung oder eine Mischungen von zwei oder mehreren Photoinitiatoren sein. Vorzugsweise ist der Photoinitiator ausgewählt aus der Gruppe bestehend aus Phosphinat, Phosphinoxid, Sulfonylketon, Sulfonylazid, der polymere Morpholinoketone, alpha-Aminoketon oder lodoniumhexafluorophosphatsalz. Vorzugsweise kann der mindestens eine Photoinitiator ausgewählt sein aus der Gruppe bestehend aus Acylphosphinoxid (z. B. 2,4,6-trimethylbenzoyl-diphenylphosphinoxid), Bisacylphosphinoxid (z.B. bis-(2,4,6-trimethylbenzoyl)-phenylphosphinoxid), Diphenylmesityloylphosphinoxid, 2,2-Dimethoxy-1,2-diphenylethan-1-on, Hydroxycyclohexylphenylketon, 2-Hydoxy-2-methylpropiophenon, 2-Benzyl-2-dimethylamino-1-(4-morpholinophenyl)-butanon-1, 2- Methyl-1 [4-(methylthio)phenyl]-2-morpholinopropan-1-on, Phenylglyoxyl-säuremethylester, 2-Hydroxy-1-{4-[4-(2-hydroxy-2-methyl-propionyl)-benzyl]-phenyl}-2-methyl-propan-1-on oder 2-Hydroxy-1-[4-(2-hydroxyethoxy)phenyl]-2-methyl-1-propanon. Bevorzugt wird erfindungsgemäß Diphenylmesityloylphosphinoxid eingesetzt, da diese Substanz durch eine hohe Reaktivität sowohl eine Erhöhung der Härtungsgeschwindigkeit als auch einen erhöhten Aushärtungsgrad bewirken kann. Der Aushärtungsgrad lässt sich nach Abschluss der Aushärtung beispielsweise durch Messung der mechanischen Eigenschaften (insbesondere Steifigkeit und Festigkeit) oder Oberflächenhärte (z.B. mit Hilfe der Bestimmung der Barcol-Härte, bei der eine federbelastete Nadel in das Testmaterial gedrückt wird) charakterisieren.

Besonders bevorzugt kann die Zusammensetzung ein strahlenhärtbares hexafunktionelles aliphatisches Polyurethan, mindestens einen ersten Ester und mindestens einen zweiten Ester umfassen, wobei der mindestens eine erste Ester idealerweise Hydroxyethylmethacrylat ist, und der mindestens eine zweite Ester idealerweise Pentaerythrittetraacrylat ist. Vorzugsweise umfasst die Zusammensetzung zusätzlich mindestens einen Photoinitiator, wobei der Photoinitiator idealerweise Pentaerythrittetraacrylat ist.

Der Photoinitiator kann beispielsweise in einer Menge von 0,1 bis 3,5 Gew.-% eingesetzt werden, bevorzugt von 0,5 bis 2,5 Gew.-%, besonders bevorzugt von 1,0 bis 2,0 Gew.-%.

In einem zweiten Aspekt betrifft die vorliegende Erfindung ein Verfahren zur kosmetischen Korrektur eines Spaltnagels, welches die folgenden Schritte umfasst: Zunächst wird auf die Nagelplatte, welche vorzugsweise gereinigt ist, die Zusammensetzung gemäß dem ersten Aspekt der Erfindung, daraufhin erfolgt die Strahlenhärtung der im vorangegangenen Schritt aufgetragenen Schicht. Das erfindungsgemäße Verfahren ist einfach durchzuführen und benötigt kein umständliches und langwieriges Aufkleben künstlicher Nägel, um einen Schutzeffekt zu erzielen. Zudem weist die Zusammensetzung eine ausreichende Polymerisationsschrumpfung auf, um eine effektive kosmetische Korrektur eines Spaltnagels durch Zusammenziehen der Risse und/oder Spalten zu ermöglichen.

In einem dritten Aspekt betrifft die vorliegende Erfindung ein Kombinationspräparat zum Aufbringen auf eine Nagelplatte. Das Kombinationspräparat umfasst dabei eine Zusammensetzung, welche mindestens ein erstes strahlenhärtbares Polyurethanacrylat, mindestens einen ersten Ester und mindestens einen zweiten Ester umfasst, wobei der erste Ester ein Methacrylatester ist, und der mindestens eine zweite Ester ein multifunktioneller Acrylatester ist, als Zusammensetzung 1, und weiterhin eine Zusammensetzung 2. Die Zusammensetzung 2 umfasst mindestens ein zweites strahlenhärtbares Polyurethanacrylat, mindestens einen multifunktionellen Polyester und mindestens einen Methacrylatester.

In einer bevorzugten Ausführungsform des erfindungsgemäßen Kombinationspräparats kann das mindestens eine zweite strahlenhärtbare Polyurethanacrylat der Zusammensetzung 2 aus der Gruppe der aliphatischen bifunktionellen Polyurethanacrylate ausgewählt sein. Vorzugsweise kann das mindestens eine zweite strahlenhärtbare Polyurethanacrylat eine dynamische Viskosität von 1.000 bis 4.500 mPa·s aufweisen. Durch die zwei funktionellen Gruppen sind die Schrumpfungseigenschaften im Vergleich mit dem Polyurethanacrylat der Zusammensetzung 1 vorteilhafterweise deutlich herabgesetzt, so dass das Polyurethanacrylat als Basiskomponente der Zusammensetzung 2 hervorragende Haftungseigenschaften auf der Nagelplatte besitzt und sich überdies gut in einer wässrigen Dispersion verarbeiten lässt. Das mindestens eine zweite strahlenhärtbare Polyurethanacrylat ist außerdem ausreichend flexibel und zudem hinreichend viskös, um ein einfaches Aufbringen auf eine Nagelplatte zu ermöglichen.

Der mindestens eine Methacrylatester der Zusammensetzung 2 des Kombinationspräparats kann ausgewählt sein aus der Gruppe bestehend aus Methylmethacrylat, Ethylmethacrylat, Propylmethacrylat, Butylmethacrylat, Hexylmethacrylat, Cyclohexylmethacrylat, Isodocylmethacrylat, Laurylmethacrylat, Stearylmethacrylat, Isobornylmethacrylat, Phenylmethacrylat, Benzylmethacrylat, Propylenmethacrylat, Glycolmethacrylat, Tetrahydrofurfurylmethacrylat, Hydroxyethylmethacrylat und Hydroxypropylmethacrylat. Idealerweise ist der mindestens eine Methacrylatester der Zusammensetzung 2 Hydroxyethylmethacrylat. Auch im Zusammenhang mit der Zusammensetzung 2 kann Hydroxyethylmethacrylat den Wasserhaushalt des Nagels vorteilhaft beeinflussen, da eine Reduktion des Nagel-Wassers unter den normalen Wassergehalt von ca. 15 Gew.-% die Brüchigkeit des Nagels infolge Austrocknung und Versprödung verstärkt.

Der multifunktionelle Polyester der Zusammensetzung 2 des Kombinationspräparats kann ausgewählt sein aus der Gruppe der durch Polykondensation von Hydroxycarbonsäuren oder Dicarbonsäuren und Polyolen, insbesondere Diolen, hergestellten Polyesteracylaten. Die Dicarbonsäure kann dabei aliphatisch, alicyclisch oder aromatisch sein, wobei den aliphatischen Säuren wegen ihrer vorteilhaften physikalischen Eigenschaften und ihrer geringeren Licht-induzierten Vergilbung der Vorzug zu geben ist. Diese Säuren können beispielsweise ausgewählt sein aus der Gruppe, sind aber nicht beschränkt auf diese: Oxalsäure, Malonsäure, Dimethylmalonsäure, Bernsteinsäure, Glutarsäure, Adipinsäure, Pimelinsäure, 2,2-Dimethylglutarsäure, Azelainsäure, Suberinsäure, Sebacinsäure, Fumarsäure, Maleinsäure, Itaconsäure, Phthalsäure, Dodecandisäure, 1,3-Cyclohexandicarbonsäure, 1,4-Cyclohexandicarbonsäure, Isophthalsäure, Terephthalsäure, 2,5-Norbornandicarbonsäure, Diglykolsäure, Thiodipropionsäure, 2,5-Naphthalindicarbonsäure und 2,6-Naphthalindicarbonsäure. Die Dicarbonsäuremonomere können allein oder in Kombination von mindestens zwei Dicarbonsäuremonomeren verwendet werden. Das Diol kann unter aliphatischen, alicylischen und aromatischen Diolen ausgewählt werden. Es kann vorzugsweise ein Diol verwendet werden, das ausgewählt ist aus Ethylenglykol, Diethylenglykol, Triethylenglykol, 1,3- Propandiol, Cyclohexandimethanol und 4-Butandiol. Weitere Polyole, die verwendet werden können, sind Glycerin, Pentaerythrit, Sorbit und Trimethylolpropan.

Besonders bevorzugt kann die Zusammensetzung 2 des Kombinationspräparats ein strahlenhärtbares bifunktionelles aliphatisches Polyurethan, mindestens einen Methacrylatester und mindestens einen multifunktionellen Polyester umfassen, wobei der Methacrylatester vorzugsweise Hydroxyethylmethacrylat ist, und der mindestens eine multifunktionelle Polyester ein niedrigvisköses Polyesteracrylat ist.

Das erfindungsgemäße Kombinationspräparat kann eine effektive kosmetische Korrektur eines Spaltnagels hervorragend ermöglichen, indem es gegenüber der Zusammensetzung 1 eine nochmals verbesserte Haftung auf der Nagelplatte aufweist. Das Kombinationspräparat ist somit ausgezeichnet geeignet, bei einer geringen Applikationshäufigkeit eine effektive kosmetische Korrektur eines Spaltnagels zu ermöglichen.

In einer weiteren Implementierung des erfindungsgemäßen Kombinationspräparats kann das mindestens eine zweite strahlenhärtbare Polyurethanacrylat der Zusammensetzung 2 in einer Konzentration von 1 bis 90 Gew.-% bezogen auf das Gesamtgewicht der Zusammensetzung in der Zusammensetzung enthalten sein. Vorzugsweise kann es im Bereich von 20 bis 85 Gew.-%, bevorzugter im Bereich von 30 bis 80 Gew.-%, insbesondere im Bereich von 40 bis 75 Gew.-% bezogen auf das Gesamtgewicht der Zusammensetzung enthalten sein. Der mindestens eine multifunktionelle Polyester kann in einer Konzentration von 1 bis 50 Gew.-% vorliegen. Vorzugsweise kann der mindestens eine multifunktionelle Polyester in einer Konzentration von 10 bis 40 Gew.-%, insbesondere von 15 bis 35 Gew.-% bezogen auf das Gesamtgewicht der Zusammensetzung vorliegen. Der mindestens eine Methacrylatester kann in einer Konzentration von 1 bis 30 Gew.-% vorliegen bezogen auf das Gesamtgewicht der Zusammensetzung. Vorzugsweise kann der mindestens eine Methacrylatester in einer Konzentration von 5 bis 25 Gew.-%, insbesondere von 10 bis 20 Gew.-% enthalten sein.

Besonders bevorzugt kann die Zusammensetzung 2 mindestens ein strahlenhärtbares Polyurethan und mindestens einen Methacrylatester umfassen, wobei das strahlenhärtbare Polyurethan in einer Konzentration von 40 bis 75 Gew.-% bezogen auf das Gesamtgewicht in der Zusammensetzung 2 enthalten ist, und der mindestens eine Methacrylatester in einer Konzentration von 10 bis 20 Gew.-% bezogen auf das Gesamtgewicht in der Zusammensetzung 2 enthalten ist. Vorzugsweise kann die Zusammensetzung 2 zusätzlich mindestens einen multifunktionellen Polyester umfassen, welcher idealerweise in einer Konzentration von 15 bis 35 Gew.-% bezogen auf das Gesamtgewicht in der Zusammensetzung 2 enthalten ist.

Durch die so definierte Zusammensetzung 2 werden die Eigenschaften der hervorragenden Aufbringbarkeit auf den Nagel vor dem Aushärten, sowie einer ausgezeichneten Festigkeit auf der Nagelplatte nach dem Aushärten miteinander verbunden.

In einem vierten Aspekt betrifft die Erfindung die Verwendung des Kombinationspräparates zur kosmetischen Korrektur eines Spaltnagels. Dabei kann die Verwendung die folgenden Schritte umfassen: in einem ersten Schritt erfolgt das Auftragen der Zusammensetzung 2 des Kombinationspräparats auf eine Nagelplatte, danach erfolgt die Strahlenhärtung der im vorangegangenen Schritt aufgetragenen Schicht. Nach ausreichender Aushärtung der Zusammensetzung 2 wird in einem nächsten Arbeitsschritt die Zusammensetzung 1 auf den Nagel aufgetragen, wiederum gefolgt von einem Strahlenhärtungs-Schritt. Optional können nach Auftrag und Aushärten der Zusammensetzung 1 weitere Schichten aufgetragen werden. Beispielsweise können die Schritte ihrer Reihenfolge nach ein- oder mehrfach wiederholt werden (Schritt (a) bis (d)). Alternativ kann nur eine einfache oder mehrfache Wiederholung der Schritte (c) und (d) erfolgen. Alternativ oder zusätzlich können beispielsweise eine oder mehrere Schicht/en eines pigmenthaltigen Lackes aufgetragen werden.

Vorzugsweise kann die Polymerisation im Wellenlängenbereich zwischen 320 und 400 nm erfolgen, wobei die Lichtintensität der Quelle: 100-3000 mW/cm² betragen kann.

Das gemäß der erfindungsgemäßen Verwendung auf den Nagel applizierte Kombinationspräparat zeichnet sich durch eine ausgezeichnete Haftung auf dem Substrat (Nagelplatte) sowie durch eine hervorragende Zwischenhaftung der Zusammensetzungen 1 und 2 aus. Beispielsweise kann die Adhäsion mittels der in ISO 8510 - 2 oder in ASTM D 903-98 festgelegten Methoden im Schälversuch bestimmt werden. Bevorzugt kann das Kombinationspräparat sich durch eine Schälfestigkeit von 0,1 und 1,5 N/25 mm, auszeichnen. (alternative/zusätzliche Prüfverfahren: Gitterschnittprüfung (ASTM D3359-07), Kratzadhäsion (ASTM D2197-98)).

Das so zu verarbeitende Kombinationspräparat zeichnet sich durch eine einfache Handhabung aus, da nur wenige Arbeitsschritte erforderlich sind. Die erfindungsgemäße Verwendung kann eine effiziente Korrektur eines Spaltnagels ermöglichen, indem der Riss oder die Risse des Spaltnagels über einen längeren Zeitraum effektiv zusammengezogen werden, beispielsweise bis ein Herauswachsen des Risses oder der Risse aus dem Nagel durch natürliches Nagelwachstum erfolgt ist. Die mechanischen Eigenschaften des erfindungsgemäßen Kombinationspräparats können eine nur geringe Applikationshäufigkeit bedingen: da ein normaler Nagel im Durchschnitt ca. 0,5 - 1,2 mm/Woche wächst (siehe: Zaun und Dill-Müller; In: Krankhafte Veränderungen des Nagels, 2004; S. 11) kann bei entsprechend kurzen Rissen und Spalten eine einzige Applikation des Kombinationspräparates ausreichen, um eine wirkungsvolle kosmetische Korrektur eines Spaltnagels zu ermöglichen. Aber auch ein Spaltnagel mit einer weit nach proximal in der Nagelplatte ausgebildeten Spaltung kann effektiv behandelt und korrigiert werden, da das Kombinationspräparat neben dem Effekt des Zusammenziehens infolge der harten Oberfläche der Zusammensetzung 1 nach der Polymerisation auch eine Schutzwirkung erzeugen kann. Dadurch kann die Gefahr zusätzlicher Verletzungen der gespaltenen Nagelplatte, beispielsweise durch umgebungsbedingte Scher- oder Zugkräftekräfte, deutlich und vorteilhaft herabgesetzt werden.

In einem fünften Aspekt betrifft die vorliegende Erfindung ein Verfahren zur kosmetischen Korrektur eines Spaltnagels, umfassend die Schritte: Auftragen der Zusammensetzung 1 des Kombinationspräparats und Strahlenhärtung der im vorangegangenen Schritt aufgetragenen Schicht, wobei die Schichtdicke von 50 µm zu 400 µm beträgt. Alternativ betrifft die die vorliegende Erfindung die Verwendung des Kombinationspräparates zur kosmetischen Korrektur eines Spaltnagels, wobei die Schichtdicke von 50 µm zu 400 µm beträgt. Ein derartig ausgeführtes Verfahren oder eine derartig ausgeführte Verwendung kann in hervorragender Weise eine kosmetische Korrektur eines Spaltnagels, ermöglichen, da die Schicht/die Schichten hinreichend dick ausgebildet ist/sind, um ein effektives Zusammenziehen von Rissen und Spalten in einer betroffenen Nagelplatte gewährleisten zu können. Gleichzeitig ist die Schichtdicke aber nur so dünn dimensioniert, dass eine gute Haftung auf der Nagelplatte auch über einen längeren Zeitraum bestehen kann. Dadurch wird die Applikationshäufigkeit des Kombinationspräparats vorteilhaft herabgesetzt, was zu einer erleichterten Anwendung und einer Kostenersparnis führt.

In einem sechsten Aspekt betrifft die vorliegende Erfindung die polymerisierte Grundmasse herstellbar aus dem Kombinationspräparat gemäß der Erfindung.

### Beispiele, kurze Beschreibung der Figuren

Im Folgenden werden experimentelle Beispiele angegeben, die die vorliegende Erfindung erläutern, ohne sie einzuschränken, sowie beispielhaft und nicht abschließend einige besondere Ausführungsformen der Erfindung unter Bezugnahme auf die beiliegenden Figuren beschrieben.

Die Darstellungen sind dabei schematisch und nicht maßstabsgetreu

### Beispiel 1

Die einzelnen Inhaltsstoffe der Zusammensetzungen 1 und 2 werden in der angegebenen Reihenfolge bei Raumtemperatur gemischt.

Zusammensetzung 1 enthält dabei Polyurethanacrylat (55%), Pentaerythrittetraacrylat (35%), Hydroxyethylmethacrylat (10%) Diphenylmesityloylphosphinoxid (2%). Zusammensetzung 2 enthält dabei Polyetherurethanacrylat (65%), Polyesteracrylat (25%) Hydroxyethylmethacrylat (10%) Diphenylmesityloylphosphinoxid (2%).

Die jeweilige Schicht wird in flüssiger Phase dünn mit einem geeigneten Applikator (Pinsel, Bürste) auf die Nagelplatte (2) aufgetragen, so dass die Schicht die Oberseite vollständig, d.h. seitlich bis zu dem Nagelfalz (6) und in der Länge von der Nagelhaut bis zu freiliegenden Nagelrand (Hyponychium), bedeckt. Die Dicke der Schicht beträgt ungefähr 100 bis 300 µm. Insbesondere das Gebiet, welches dem Riss/Nagelspalt oder den Rissen/Nagelspalten benachbart ist, wird in bezüglich der Nagelplatte längs- und querverlaufender Richtung über den Riss/Nagelspalt oder die Risse/Nagelspalten hinweg mit der jeweiligen Zusammensetzung bedeckt, so dass in diesem Bereich die Schichtdicke näher am oberen Wert von ungefähr 300 µm gelegen ist.

Die Zusammensetzung wird verfestigt, indem die aufgetragene Schicht mit einer geeigneten UV-Lampe über einen Zeitraum von 1 min. (Zusammensetzung 1) bzw. 30 sec. (Zusammensetzung 2) bestrahlt wird, wobei sich die Schicht erhärtet. Geeignete UV Lampen sind dabei beispielsweise herkömmliche, in einem Nagelstudio verwendete UV Röhrenlampen mit einer Leistung von 36 bis 60 Watt, vorzugsweise können aber Hochleistungs - UV Lampen, beispielsweise Hochleistungs - UV LED Lampen mit einer Leistung von größer als 54 Watt, zum Einsatz kommen. Die Bestrahlung erfolgt im Wellenlängenbereich von 250 bis 450 nm, vorzugsweise im Bereich von 350 bis 400 nm.

Die in einer ersten Schicht aufgetragene Zusammensetzung 2 wirkt dabei als Bonder und dient der festeren Verbindung der in einem zweiten Schritt aufgetragenen Zusammensetzung 1 mit der Nagelplatte. Die zweite Schicht kontrahiert sich, so dass Kräfte Fₓ über den Riss/Nagelspalt oder die Risse/Nagelspalten hinweg in Richtung der Nagelplattenmitte wirken und damit ein Zusammenziehen des Risses/Nagelspalts oder der Risse/Nagelspalten bewirken. Die Nagelplatte verändert sich also unter den Bedingungen hinsichtlich der Dimensionierung: es findet eine Kontraktion statt, welche insbesondere im Bereich von Defekten der Nagelplatte zu einem Zusammenziehen der Defekt-Ränder führt, so dass eine Korrektur des Nagels erreicht wird.

### Beispiel 2, Messverfahren:

Zum Einsatz kommen entweder Nagelabschnitte von Versuchspersonen, Abschnitte künstlicher Nägel oder geeignete künstliche Nägel. Zunächst wird der Nagelabschnitt angeschliffen, gereinigt, auf einem herkömmlichen Millimeterpapier fotografiert (Schritt 1) und anschließend für drei Stunden zur Simulation des natürlichen Nagelbetts auf feuchtem Ton gelagert (Schritt 2). Ein weiteres Foto wird aufgenommen (Schritt 3). Eine Schicht einer ersten Zusammensetzung, beispielsweise eine Schicht eines Bonders, wird aufgetragen und durch Bestrahlen mit einem UV Strahler bei einer Wellenlänge von 250 bis 450 nm, einer Leistung von 100 bis 3000 mW/cm² für 30 bis 120 sec. gehärtet (Schritt 4). An die UV Härtung schließt sich eine Abkühlzeit von 30 bis 150 sec. an, die der Auspolymerisation dient (Schritt 5). Eine weitere Schicht einer zweiten Zusammensetzung wird aufgetragen, beispielsweise eine Schicht einer Formulierung, welche einen Polymerisationsschrumpf aufweist, und durch Bestrahlen mit einem UV Strahler bei einer Wellenlänge von 250 bis 450 nm, einer Leistung von 100 bis 3000 mW/cm² für 30 bis 120 sec. gehärtet (Schritt 6). Nach dem Abkühlen (Aushärten) für mindestens 30 sec. (Schritt 7) wird der Nagelabschnitt gereinigt und wiederum auf einem herkömmlichen Millimeterpapier fotografiert (Schritt 8). Nach dem Fotografieren wird der Nagelabschnitt auf feuchten Ton transferiert (Schritt 9). Nach einer weiteren Stunde auf feuchtem Ton wird der Nagelabschnitt nochmals einem herkömmlichen Millimeterpapier fotografiert (Schritt 10).

Optional kann das Messverfahren auch nur das Auftragen einer Schicht umfassen, so dass nach Schritt 4 mit Schritt 7 fortgefahren wird.

### Figuren

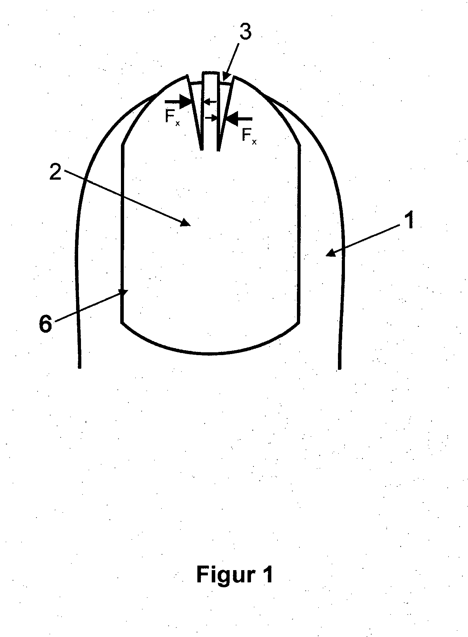

Fig. 1 zeigt eine Draufsicht auf einen Spaltnagel eines Fingers (1). Dargestellt sind eine Nagelplatte (2) mit zwei nach distal ausgedehnten Spalten (3), die Nagelfalz (6), sowie die nach dem Auftragen der kontrahierenden Schicht (nicht dargestellt) im Bereich der Spalten wirkenden Kräfte, Fₓ. Die Kräfte Fₓ wirken in der Schichtebene über die Ränder der Spalten hinweg und ziehen diese zusammen.

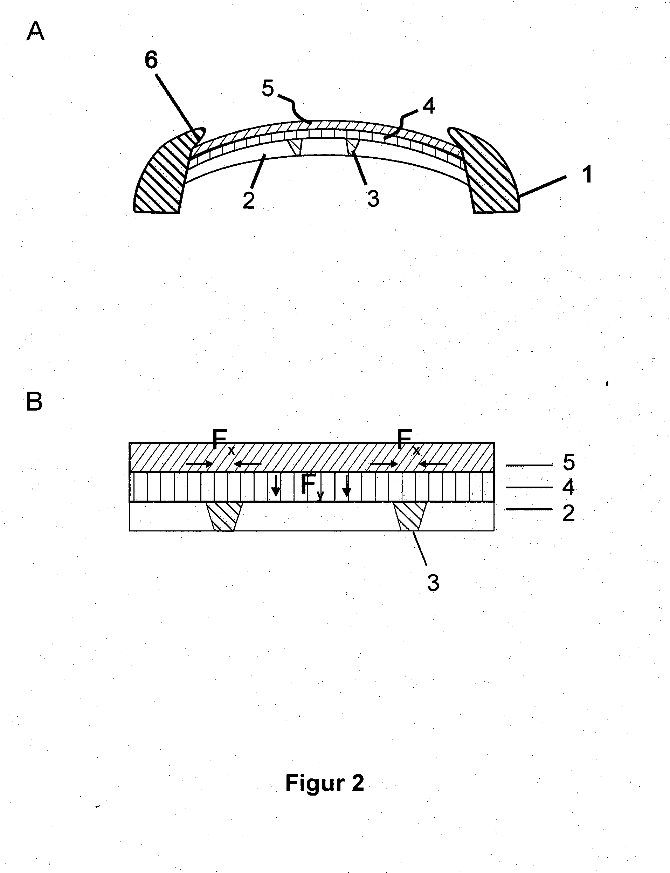

Fig. 2A. zeigt einen Spaltnagel im Querschnitt (nicht maßstabsgerecht) mit zwei in der Nagelplatte (2) angeordneten Spalten (3). Auf die Nagelplatte (2) sind zwei Schichten (4,5) aufgetragen, wobei die der Nagelplatte (2) zugewandte erste Schicht (4) als Bonderschicht fungiert. Im Rahmen der vorliegenden Erfindung kann die Bonderschicht vorteilhaft aus der Zusammensetzung 2 bestehen. Auf die erste Schicht (4) kann nach deren Aushärtung eine zweite Schicht (5) aufgebracht werden, welche im Rahmen der vorliegenden Erfindung vorteilhaft aus der Zusammensetzung 1 bestehen kann. In Figur 2B sind die Zugrichtungen schematisch dargestellt, welche sich vorteilhaft auf die Nagelplatte (2) und die darin ausgebildeten Spalten (3) übertragen. Die Bonderschicht wirkt mit der Kraft Fy in Richtung Nagelplatte (2). Die zweite Schicht (5) dient dem Zusammenziehen der Spalte/der Spalten (3) und weist eine über die jeweilige Spalte (3) gerichtete Zugrichtung Fₓ auf, welche aufgrund der Polymerisationsschrumpfung entsteht und welche vor allem innerhalb der zweiten Schicht (5) wirkt. Der jeweilige Spalt (3) wird wegen seiner im Vergleich zur Nagelplatte (2) geringer ausgebildeten Festigkeit infolge der dadurch vermehrten Wirksamkeit der Polymerisationsschrumpfung zusammengezogen.

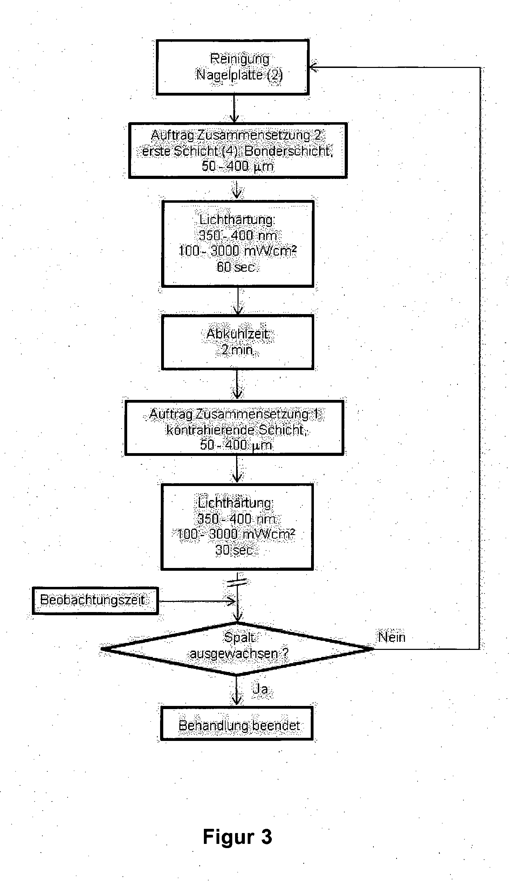

Fig. 3 zeigt ein Fluss-Diagramm des Verfahrens zur Korrektur eines Spaltnagels (1) unter Verwendung von zwei Schichten (4,5). Dabei erfolgt zunächst die Reinigung der Nagelplatte (2). Auf die gesäuberte Nagelplatte wird in einer ersten Schicht (4) die Zusammensetzung 2, entsprechend der Bonderschicht, aufgetragen (Schichtdicke: 100-300 µm), gefolgt von einem UV Härtungsschritt für 1 min. bei einer Leistung von 100-3000 mW/cm² und einer Wellenlänge von 350 bis 400 nm. Nach einer 2 minütigen Abkühlzeit, welche der Auspolymerisierung dient erfolgt der Auftrag der Zusammensetzung 1, der kontrahierenden Schicht. An diesen Schritt schließt sich wiederum ein UV Härtungsschritt an, in diesem Fall von einer Dauer für 30 sec. bei einer Leistung von 100-3000 mW/cm² und einer Wellenlänge von 350 bis 400 nm. In folgenden Beobachtungszeitraum wird das Auswachsen des Spaltes infolge des natürlichen Nagelwachstums abgewartet, bzw. bei sehr weit nach proximal reichenden und/oder sehr tiefliegenden Spalten oder Rissen wird die Behandlung wiederholt, je nach vorliegendem Befund bis zum endgültigen Herauswachsen des Risses.

### Bezugszeichen liste

- 1: Nagel eines Fingers
- 2: Nagelplatte
- 3: Nagelspalt
- 4: erste Schicht, z. B. Bonderschicht
- 5: zweite Schicht, z. B. kontrahierende Schicht
- 6: Nagelfalz

## Patentansprüche

1. Zusammensetzung, umfassend mindestens ein erstes strahlenhärtbares Polyurethanacrylat, mindestens einen ersten Ester, wobei der erste Ester ein Methacrylatester ist, und mindestens einen zweiten Ester, wobei der zweite Ester ein multifunktioneller Acrylatester ist, und
wobei die Zusammensetzung eine Polymerisationsschrumpfung gemessen nach 24 Stunden von größer oder gleich 2 %, vorzugsweise größer oder gleich 4 % und insbesondere größer oder gleich 5 % aufweist.

2. Zusammensetzung gemäß Anspruch 1, wobei die Zusammensetzung das mindestens eine erste strahlenhärtbare Polyurethanacrylat in einer Konzentration von 1 bis 80 Gew.-%, vorzugsweise von 10 bis 70 Gew.-%, bevorzugter von 20 bis 65 Gew.-% und insbesondere von 30 bis 60 Gew.-% bezogen auf das Gesamtgewicht der Zusammensetzung aufweist.

3. Zusammensetzung gemäß einem der Ansprüche 1 oder 2, wobei die Zusammensetzung den mindestens einen ersten Ester in einer Konzentration von 1 bis 30 Gew.-%, vorzugsweise von 5 bis 25 Gew.-%, insbesondere von 10 bis 20 Gew.-% bezogen auf das Gesamtgewicht der Zusammensetzung aufweist und den mindestens einen multifunktionellen Acrylatester in einer Konzentration von 1 bis 50 Gew.-%, vorzugsweise von 5 bis 45 Gew.-% und insbesondere von 10 bis 40 Gew.-% bezogen auf das Gesamtgewicht der Zusammensetzung aufweist.

4. Zusammensetzung gemäß einem der vorangehenden Ansprüche, wobei das mindestens eine erste strahlenhärtbare Polyurethanacrylat aus der Gruppe der aliphatischen multifunktionellen Polyurethanacrylate ausgewählt ist, und wobei das mindestens eine erste strahlenhärtbare Polyurethanacrylat eine dynamische Viskosität von 100 bis 2.000 mPa·s aufweist.

5. Zusammensetzung gemäß einem der vorangehenden Ansprüche, zusätzlich umfassend mindestens einen Photoinitiator.

6. Verfahren zur kosmetischen Korrektur eines Spaltnagels, umfassend die folgenden Schritte:
(a) Auftragen der Zusammensetzung gemäß einem der Ansprüche 1 bis 6,
(b) Strahlenhärtung der in Schritt (a) aufgetragenen Schicht.

7. Kombinationspräparat zum Aufbringen auf eine Nagelplatte, umfassend mindestens eine Zusammensetzung 1 und eine Zusammensetzung 2, wobei die Zusammensetzung 1 mindestens ein erstes strahlenhärtbares Polyurethanacrylat, mindestens einen ersten Ester und mindestens einen zweiten Ester umfasst, wobei der mindestens eine erste Ester ein Methacrylatester ist und wobei der mindestens eine zweite Ester ein multifunktioneller Acrylatester ist, und
wobei die Zusammensetzung 2 mindestens ein zweites strahlenhärtbares Polyurethanacrylat, mindestens einen multifunktionellen Polyester und mindestens einen Methacrylatester umfasst.

8. Kombinationspräparat gemäß Anspruch 7, wobei die Zusammensetzung 1 die in den Ansprüchen 1 bis 6 definierte Zusammensetzung ist.

9. Kombinationspräparat gemäß Anspruch 7 oder 8, wobei die Zusammensetzung das mindestens eine zweite strahlenhärtbare Polyurethanacrylat in einer Konzentration von 1 bis 90 Gew.-%, vorzugsweise von 20 bis 85 Gew.-%, bevorzugter von 30 bis 80 Gew.-%, insbesondere von 40 bis 75 Gew.-% bezogen auf das Gesamtgewicht der Zusammensetzung umfasst.

10. Kombinationspräparat gemäß einem der Ansprüche 7 bis 9, wobei die Zusammensetzung den mindestens einen multifunktionellen Polyester in einer Konzentration von 1 bis 50 Gew.-%, vorzugsweise von 10 bis 40 Gew.-%, insbesondere von 15 bis 35 Gew.-% bezogen auf das Gesamtgewicht der Zusammensetzung umfasst und den mindestens einen zweiten Methacrylatester in einer Konzentration von 1 bis 30 Gew.-%, vorzugsweise von 5 bis 25 Gew.-%, insbesondere von 10 bis 20 Gew.-% bezogen auf das Gesamtgewicht der Zusammensetzung umfasst.

11. Kombinationspräparat gemäß einem der Ansprüche 7 bis 10, wobei das mindestens eine zweite strahlenhärtbare Polyurethanacrylat aus der Gruppe der aliphatischen bifunktionellen Polyurethanacrylate ausgewählt ist, und wobei das mindestens eine zweite strahlenhärtbare Polyurethanacrylat eine dynamische Viskosität von 1.000 bis 4.500 mPa·s aufweist.

12. Verwendung des Kombinationspräparates gemäß einem der Ansprüche 7 bis 11 zur kosmetischen Korrektur eines Spaltnagels.

13. Verwendung des Kombinationspräparates gemäß Anspruch 12, umfassend die folgenden Schritte:
(a) Auftragen der Zusammensetzung 2 des Kombinationspräparats auf einen Nagel,
(b) Strahlenhärtung der in Schritt (a) aufgetragenen Schicht,
(c) Auftragen der Zusammensetzung 1 auf den Nagel, und
(d) Strahlenhärtung in Schritt (c) aufgetragenen Schicht.

14. Verfahren gemäß Anspruch 6 oder Verwendung gemäß einem der Ansprüche 12 oder 13, wobei die Schichtdicke von 50 µm bis zu 400 µm beträgt.

15. Polymerisierte Grundmasse herstellbar aus dem Kombinationspräparat gemäß einem der Ansprüche 7 bis 11.

## Claims

1. Composition, comprising at least one radiation-curable polyurethane acrylate, at least one first ester, wherein the first ester is a methacrylate ester, and at least one second ester, wherein the second ester is a multifunctional acrylic ester, and wherein the composition is **characterized by** a polymerization shrinkage measured after 24 hours of greater than or equal to 2%, preferably greater than or equal to 4% and in particular greater than or equal to 5%.

2. Composition according to claim 1, wherein the at least one radiation-curable polyurethane acrylate in the composition has a concentration of from 1 to 80 wt%, preferably from 10 to 70 wt%, more preferably from 20 to 65 wt%, and in particular from 30 to 60 wt%, based on the total weight of the composition.

3. Composition according to any one of claims 1 or 2, wherein the at least one first ester in the composition has a concentration of from 1 to 30 wt%, preferably from 5 to 25 wt%, in particular from 10 to 20 wt%, based on the total weight of the composition, and wherein the at least one multifunctional acrylic ester in the composition has a concentration of from 1 to 50 wt %, preferably from 5 to 45 wt% and in particular from 10 to 40 wt%, based on the total weight of the composition.

4. Composition according to any one of the preceding claims, wherein the at least one first radiation-curable polyurethane acrylate is selected from the group of aliphatic multifunctional polyurethane acrylates, and wherein the at least one first radiation-curable polyurethane acrylate exhibits a dynamic viscosity of from 100 to 2.000 mPa•s.

5. Composition according to any one of the preceding claims, additionally comprising at least one photoinitiator.

6. Method for cosmetically correcting a split-nail, comprising the following steps:
(a) applying the composition according to any one of the claims 1 to 6,
(b) curing by radiation of the composition applied in step (a).

7. Combination preparation for application onto a nail-plate, comprising at least one composition 1 and one composition 2, wherein composition 1 comprises at least one first radiation-curable polyurethane acrylate, at least one first ester and at least one second ester, wherein the at least one first ester is a methacrylate ester and wherein the at least one second ester is a multifunctional acrylic ester, and
wherein composition 2 comprises at least one second radiation-curable polyurethane acrylate, at least one multifunctional polyester and at least one methacrylate ester.

8. Combination preparation according to claim 7, wherein composition 1 is the composition according to any one of the claims 1 to 6.

9. Combination preparation according to claim 7 or 8, wherein the at least one second radiation-curable polyurethane acrylate in the composition has a concentration of from 1 to 90 wt%, preferably from 20 to 85 wt%, more preferably from 30 to 80 wt%, in particular from 40 to 75 wt%, based on the total weight of the composition.

10. Combination preparation according to any one of claims 7 to 9, wherein the at least one multifunctional polyester in the composition has a concentration of from 1 to 50 wt%, preferably from 10 to 40 wt%, in particular from 15 to 35 wt%, based on the total weight of the composition, and the at least one second methacrylate ester has a concentration of from 1 to 30 wt%, preferably from 5 to 25 wt%, in particular from 10 to 20 wt%, based on the total weight of the composition.

11. Combination preparation according to any one of claims 7 to 10, wherein the at least one second radiation-curable polyurethane acrylate is selected from the group of aliphatic bi-functional polyurethane acrylates, and wherein the at least one second radiation-curable polyurethane acrylate exhibits a dynamic viscosity of from 1000 to 4500 mPa•s.

12. Use of the combination preparation according to any one of claims 7 to 11 for the cosmetic correction of a split nail.

13. Use of the combination preparation according to claim 12, comprising the steps of:
(a) applying composition 2 of the combination preparation onto a nail,
(b) curing by radiation of the layer applied in step (a),
(c) applying composition 1 onto the nail, and
(d) curing by radiation of the layer applied in step (c).

14. Method according to claim 6 or use according to any one of claims 12 or 13, wherein the thickness of the layer amounts to 50 µm to 400 µm.

15. Polymerized matrix producible from the combination preparation according to any one of claims 7 to 11.

## Revendications

1. Composition, contenant au moins un premier polyuréthanacrylate durcissable par rayonnement, au moins un premier ester, le premier ester étant un ester de méthacrylate, et au moins un deuxième ester, le deuxième ester étant un ester d'acrylate multifonctionnel, et
ladite composition présentant un retrait de polymérisation mesuré après 24 heures supérieur ou égal à 2 %, préférentiellement supérieur ou égal à 4 % et tout particulièrement supérieur ou égal à 5 %.

2. Composition selon la revendication 1, où ladite composition contient le ou les premiers polyuréthanacrylates durcissables par rayonnement dans une concentration de 1 à 80 % en poids, avantageusement de 10 à 70 % en poids, préférentiellement de 20 à 65 % en poids et tout particulièrement de 30 à 60 % en poids par rapport au poids total de la composition.

3. Composition selon la revendication 1 ou la revendication 2, où ladite composition contient le ou les premiers esters dans une concentration de 1 à 30 % en poids, préférentiellement de 5 à 25 % en poids, tout particulièrement de 10 à 20 % en poids par rapport au poids total de la composition, et où le ou les esters d'acrylate multifonctionnels dans une concentration de 1 à 50 % en poids, préférentiellement de 5 à 45 % en poids et tout particulièrement de 10 à 40 % en poids par rapport au poids total de la composition.

4. Composition selon l'une des revendications précédentes, où le ou les premiers polyuréthanacrylates durcissables par rayonnement sont sélectionnés dans le groupe des polyuréthanacrylates aliphatiques multifonctionnels, et où le ou les premiers polyuréthanacrylates durcissables par rayonnement présentent une viscosité dynamique comprise entre 100 et 2.000 mPa•s.

5. Composition selon l'une des revendications précédentes, contenant en outre au moins un photoinitiateur.

6. Procédé de correction cosmétique d'un ongle fendu, comprenant les étapes suivantes :
(a) application de la composition selon l'une des revendications 1 à 6,
(b) durcissement par rayonnement de la couche appliquée en étape (a).

7. Préparation combinée destinée à être appliquée sur un corps d'ongle, comprenant au moins une composition 1 et une composition 2, la composition 1 contenant au moins un premier polyuréthanacrylate durcissable par rayonnement, au moins un premier ester et au moins un deuxième ester, chaque premier ester étant un ester de méthacrylate et chaque deuxième ester étant un ester d'acrylate multifonctionnel, et
la composition 2 contenant au moins un deuxièmes polyuréthanacrylate durcissable par rayonnement, au moins un polyester multifonctionnel et au moins un ester de méthacrylate.

8. Préparation combinée selon la revendication 7, où la composition 1 est la composition définie en revendications 1 à 6.

9. Préparation combinée selon la revendication 7 ou la revendication 8, où la composition contient le ou les deuxièmes polyuréthanacrylates durcissables par rayonnement dans une concentration de 1 à 90 % en poids, avantageusement de 20 à 85 % en poids, préférentiellement de 30 à 80 % en poids, tout particulièrement de 40 à 75 % en poids par rapport au poids total de la composition.

10. Préparation combinée selon l'une des revendications 7 à 9, où la composition contient le ou les polyesters multifonctionnels dans une concentration de 1 à 50 % en poids, préférentiellement de 10 à 40 % en poids, tout particulièrement de 15 à 35 % en poids par rapport au poids total de la composition, et le ou les deuxièmes esters de méthacrylate dans une concentration de 1 à 30 % en poids, préférentiellement de 5 à 25 % en poids, tout particulièrement de 10 à 20 % en poids par rapport au poids total de la composition.

11. Préparation combinée selon l'une des revendications 7 à 10, où le ou les deuxièmes polyuréthanacrylates durcissables par rayonnement sont sélectionnés dans le groupe des polyuréthanacrylates aliphatiques bifonctionnels, et où le ou les deuxièmes polyuréthanacrylates durcissables par rayonnement présentent une viscosité dynamique comprise entre 1.000 et 4.500 mPa•s.

12. Utilisation de la préparation combinée selon l'une des revendications 7 à 11 pour la correction cosmétique d'un ongle fendu.

13. Utilisation de la préparation combinée selon la revendication 12, comprenant les étapes suivantes :
(a) application de la composition 2 de la préparation combinée sur un ongle,
(b) durcissement par rayonnement de la couche appliquée en étape (a),
(c) application de la composition 1 sur l'ongle, et
(d) durcissement par rayonnement de la couche appliquée en étape (c).

14. Procédé selon la revendication 6 ou utilisation selon l'une des revendications 12 ou 13, où l'épaisseur de couche est comprise entre 50 µm et 400 µm.

15. Masse polymérisée pouvant être produite à partir de la préparation combinée selon l'une des revendications 7 à 11.
